# EUROPÄISCHE PATENTSCHRIFT

(11) **EP 0 875 236 B1**
(45) Veröffentlichungstag und Bekanntmachung des Hinweises auf die Patenterteilung: **26.10.2005**
(21) Anmeldenummer: 98107117.8
(22) Anmeldetag: 20.04.1998
(51) Int. Cl.: A61K 7/06, A61K 7/50

(54) **Verwendung einer wässrigen Zubereitung zur Behandlung keratinischer Fasern**
Use of an aqueous composition for treating keratinous fibers
Utilisation d'une composition aqueuse traitante pour les fibres kératiniques

(30) Priorität: 28.04.1997 DE 19717687
(43) Veröffentlichungstag der Anmeldung: 04.11.1998
(73) Patentinhaber: Hans Schwarzkopf & Henkel GmbH & Co. KG, 22763 Hamburg (DE)
(72) Erfinder: Seidel, Winfried, Dr. Dipl.-Chem., 25451 Quickborn (DE); Goddinger, Dieter, Dr. Dipl.-Chem., 25436 Tornesch (DE); Meyer, Jens, 22880 Wedel (DE); Foitzik, Joachim Kurt, Dr. Dipl.-Ing., 64342 Seeheim-Jugenheim (DE)
(74) Vertreter: Strohe-Kamp, Geertje

(56) Entgegenhaltungen:
- EP-A- 0 733 357
- WO-A-92/09677
- FR-A- 2 751 541
- GB-A- 2 280 906
- US-A- 4 678 603
- A. HUNTING: "Encyclopedia of shampoo ingredients, 2nd edition" 1987 , MICELLE PRESS , USA XP002136303 Tabelle C1: Zusatzstoffe 1-121; 194-235 * Seite 235 - Seite 236 *

## Beschreibung

Die Erfindung betrifft die Verwendung wäßriger Zubereitungen zur Behandlung von keratinischen Fasern, insbesondere Haaren, mit speziellen Parfümölen und speziellen Bitterstoffen.

Die Reinigung und Pflege der Haare sowie die dekorative Gestaltung der Frisur sind wichtige Bestandteile der menschlichen Körperpflege. Entsprechend groß sind die Bemühungen, sowohl dem Friseur als auch dem Endverbraucher in jeder Hinsicht optimierte Produkte zur Verfügung zu stellen. Ein Schwerpunkt der Entwicklungsarbeit liegt dabei selbstverständlich bei den eigentlichen Produkteigenschaften, sei es die Reinigungswirkung eines Shampoos, der Repair-Effekt einer Haarkur, die Färbeeigenschaften eines Färbemittels oder einer Tönung, die Qualität einer Dauerwelle oder der Frisurenhalt eines Festigers oder Haarsprays. Gleichzeitig wird auch versucht, möglichen unerwünschten Nebenwirkungen, wie sie bei bestimmten Verbrauchergruppen wie beispielsweise Allergikern oder sehr empfindlichen Personen auftreten können, in immer höherem Maße vorzubeugen. Dies führte beispielsweise zur Entwicklung besonders hautfreundlicher Tensidsysteme für reinigende Mittel und zu Färbe- und Dauerwellmitteln, die in ihrem pH-Wert in der Nähe des Neutralpunktes liegen bzw. dem pH-Wert menschlicher Haut möglichst nahe kommen. Ein weiterer Punkt ist die Steigerung der Öko-Verträglichkeit der Produkte, beispielsweise durch Entwicklung besonders gut abbaubarer Tenside und die Verwendung natürlicher insbesondere nachwachsender Rohstoffe in den Formulierungsgrundlagen.

Schließlich stellt der Verbraucher in unserer modernen Konsumgesellschaft ständig steigende Anforderungen auch hinsichtlich der Konfektionierung der angebotenen Produkte. Insbesondere, wenn eine Reihe von Produkten mit vergleichbaren Eigenschaften zur Auswahl stehen, wird der Konsument sich in seiner Kaufentscheidung nicht unwesentlich von ästhetischen Gesichtspunkten wie dem optischen Erscheinungsbild oder dem Duft des Produktes leiten lassen. Dies schließt einerseits den Fall ein, daß der Verbraucher den "Erlebniswert" einer bewußt vorgenommenen, eine längere Zeit beanspruchenden (z.B. einer Dauerwelle oder einer permanenten Haarfärbung) oder häufig wiederkehrenden (z.B. Haarwäsche) Handlung durch einen angenehmen Duft erhöhen will. Zu nennen ist aber insbesondere auch die Möglichkeit, die Erziehung von Kindern zu einer adäquaten Körperpflege - und hier stellt die Haarwäsche in vielen Fällen ein besonderes Problem dar - durch Signale und Erlebnisse, wie sie bestimmte Düfte vermitteln können, zu unterstützen.

Nicht zuletzt ist aber auch die Produktsicherheit, sei es beim bestimmungsgemäßen oder nicht bestimmungsgemäßen Gebrauch, ein Kriterium, das seit einigen Jahren bei vielen Verbrauchern immer mehr von zentraler Bedeutung für ihre Kaufentscheidung wird. So waren zum Beispiel in den 80er Jahren Shampoos und Badezusätze mit starker Parfümierung "grüner Apfel" in der Meinung der Verbraucher unproblematisch und wurden gerne gekauft. Mit, nicht zuletzt durch entsprechend aufgemachte Medienberichterstattung, gewachsenem Mißtrauen, sei es in der jeweiligen Sache nun begründet oder nicht, wird er sich heute zunehmend auch Gedanken hinsichtlich eines denkbaren, noch so unwahrscheinlichen Gebrauchs des Produktes machen. So wird er es nicht ausschließen, daß zum Beispiel Produktspritzer im Bad das Interesse von Kleinkindern erwecken und von diesen im Rahmen ihres Erkundungstriebes probiert werden. Auch wenn dieses "Probieren" aufgrund des Ungiftigkeit der Produkte unschädlich ist, wird es der verantwortungsbewußte, gleichwohl nichtfachmännische Verbraucher als subjektive Erhöhung des Produktsicherheit und positive erzieherische Maßnahme ansehen und honorieren, wenn einem Herunterschlucken des Mittels prinzipiell vorgebeugt wird. Dies kann durch Zugabe von speziell dafür zugelassenen Bitterstoffen erreicht werden, die auch akzidentelle Ingestionen mit hoher Zuverlässigkeit zu verhindern.

Insbesondere wenn die entsprechenden kosmetischen Produkte Inhaltsstoffe enthalten, die den Benutzer an bekannte Lebens- oder Genußmittel erinnern, besteht die Aufgabe, solche Bitterstoffe auszuwählen, die einerseits sicher und in sehr niedriger Konzentration der ungewünschten Ingestion vorbeugen, andererseits aber nicht durch Eigenduft oder Wechselwirkung mit einzelnen Parfümölkomponenten zu einer vom Verbraucher wahrgenommenen, unerwünschten Verschiebung der Duftnote führen.
Weiterhin besteht die Aufgabe, solche Bitterstoffe auszuwählen, die sich einfach und reproduzierbar in die Produkte einarbeiten lassen. Eng verknüpft damit ist das Problem, daß diese Einarbeitung mit keinerlei negativen Auswirkungen für die mit der Herstellung beschäftigten Mitarbeiter führen darf.

Weiterhin ist es erwünscht, daß sich diese Bitterstoffe auch für solche Kosmetika eignen, die hinsichtlich der anderen oben genannten Parameter wie Mildheit, Ökoverträglichkeit und selbstverständlich Produkteigenschaften möglichst optimal zusammengesetzt sind.

Es wurde nun gefunden, daß Bitterstoffe, die bestimmte Parameter erfüllen, in hervorragender Weise zur Lösung der oben genannten Aufgaben und zur Herstellung von Produkten mit den genannten Eigenschaften geeignet sind.

Gegenstand der Erfindung ist daher die Verwendung einer wäßrigen Zubereitung zur Behandlung von Keratinfasern, insbesondere menschlichen Haaren, enthaltend mindestens einen natürlichen oder synthetischen Inhaltsstoff mit der Duftnote eines Lebens- oder Genußmittels, dadurch gekennzeichnet, daß die Zubereitung weiterhin 0,0005 bis 0,1 Gew.-% eines Bitterstoffes enthält, der eine Molmasse von mindestens 250 g/mol aufweist und in der Zubereitung bei 20 °C zu mindestens 10 mg/l löslich ist.

Da gefunden wurde, daß die gewünschten Eigenschaften des Bitterstoffes besonders schnell zur Entfaltung kommen, wenn dieser in gelöster Form in einem mindestens teilweise aus Wasser bestehenden Lösungsmittel vorliegt, ist es erfindungsgemäß bevorzugt, daß die Gesamtmenge des Bitterstoffes in den wäßrigen Zubereitungen gelöst vorliegt.

Weiterhin hat es sich als vorteilhaft erwiesen, den Bitterstoff in gelöster Form in die erfindungsgemäßen Zubereitungen einzuarbeiten, wobei sich für die exakte Dosierung die wäßrige Lösung als besonders geeignet erwiesen hat. Daher sind erfindungsgemäß Bitterstoffe bevorzugt, die in Wasser bei 20 °C zu mindestens 5 g/l löslich sind. In Abhängigkeit von dem jeweiligen Bitterstoff ist es selbstverständlich aber auch möglich, die Bitterstoffe gelöst in einer anderen Komponente der Zubereitung bereitzustellen und in dieser Form in die Zubereitung einzuarbeiten. Beispiele für weitere geeignete Lösungsmittel sind Ethanol, Isopropanol, Monoethylenglykol, Diethylenglykol, Triethylenglykol, Dimethylether und Glycerin.

Hinsichtlich einer unerwünschten Wechselwirkung mit den Duft-Komponenten, insbesondere einer Veränderung der vom Verbraucher wahrgenommenen Duftnote, haben die ionogenen Bitterstoffe sich den nichtionogenen als überlegen erwiesen. Ionogene Bitterstoffe, bevorzugt bestehend aus organischem(n) Kation(en) und organischem(n) Anion(en), sind daher für die erfindungsgemäßen Zubereitungen bevorzugt.

Erfindungsgemäß hervorragend geeignet als Bitterstoffe sind quartäre Ammoniumverbindungen, die sowohl im Kation als auch im Anion eine aromatische Gruppe enthalten. Eine solche Verbindung ist das kommerziell z.B. unter den Warenzeichen Bitrex® und Indigestin® erhältliche Benzyldiethyl((2,6-Xylylcarbamoyl)methyl)ammoniumbenzoat. Diese Verbindung ist auch unter der Bezeichnung Denatonium Benzoate bekannt.

Der Bitterstoff ist in den erfindungsgemäß verwendeten wäßrigen Zusammensetzungen in Mengen von 0,0005 bis 0,1 Gew.-%, bezogen auf die gesamte Zubereitung, enthalten. Besonders bevorzugt sind Mengen von 0,001 bis 0,05 Gew.-%.

Die erfindungsgemäß verwendeten Zubereitungen enthalten weiterhin als zwingende Komponente einen Bestandteil mit der Duftnote eines Lebens- oder Genußmittels. Dabei kann es sich um einen Bestandteil handeln, der sowohl als Wirkstoff dient und als auch die Geruchsnote entfaltet. Erfindungsgemäß bevorzugt ist aber, daß es sich bei diesem Bestandteil um ein Parfümöl handelt.

In diesem Zusammenhang soll nochmals darauf hingewiesen werden, daß dieser Bestandteil der Zubereitung den Duft eines Lebens- oder Genußmittels verleiht; dies ist nicht zwangsläufig damit verbunden, daß dadurch der Zubereitung auch der Geschmack des entsprechenden Lebens- oder Genußmittels verliehen wird. Gemäß der bevorzugten Ausführungsform der vorliegenden Erfindung verleiht dieser Bestandteil der Zubereitung ausschließlich den Duft des Lebens- oder Genußmittels; der Geschmack der Zubereitung wird durch diese Komponente zumindest nicht in Richtung des Lebens- oder Genußmittels, bevorzugt überhaupt nicht, beeinflußt.

Gemäß einer ersten bevorzugten Ausführungsform der Erfindung enthält die Zubereitung ein Parfümöl mit der Duftnote einer Frucht. Bevorzugt sind die Duftnoten der folgender Früchte: Apfel, Birne, Erdbeere, Pfirsich, Aprikose, Ananas, Banane, Kirsche, Kiwi, Mango, Kokos, Mandel, Grapefruit, Maracuja, Mandarine und Melone. Die Duftnoten von Apfel, Erdbeere und Pfirsich sind besonders bevorzugt.

Gemäß einer zweiten bevorzugten Ausführungsform der Erfindung enthält die Zubereitung ein Parfümöl mit der Duftnote eines Genußmittels. Innerhalb dieser Ausführungsform sind folgende Duftnoten bevorzugt: Tabak, Cola, Guarana, Schokolade, Kakao, Sarsaparilla, Pfefferminze und Rum.

Diese Parfümöle sind in den erfindungsgemäßen Zubereitungen bevorzugt in Mengen von 0,1 Gew.-% bis 1,0 Gew.-%, bezogen auf die gesamte Zubereitung, enthalten.

Unter Keratinfasern werden erfindungsgemäß Pelze, Wolle, Federn und insbesondere menschliche Haare verstanden.

Neben den beiden genannten, zwingenden Inhaltsstoffen und Wasser können die erfindungsgemäß verwendeten Zubereitungen noch weitere Komponenten, insbesondere übliche kosmetische Bestandteile, enthalten. Die Auswahl dieser weiteren Komponenten wird der Fachmann entsprechend des Typs des zu formulierenden Haarbehandlungsmittels vornehmen. Dieser Typ unterliegt keinen prinzipiellen Einschränkungen, so daß es sich beispielsweise um Shampoos Haarspülungen, Haarkuren, Dauerwellmittel (Wellotionen, Fixiermittel), Haarfärbemittel, Haartönungsmittel, Haarfestiger, Haarsprays, Haarwässer oder Haarspitzenfluids handeln kann Entsprechend dem Verwendungszweck können die Zubereitungen als Lösungen, Emulsionen, Gele, Cremes, Aerosole oder Lotionen formuliert werden.

Gemäß einer bevorzugten Ausführungsform handelt es sich bei den erfindungsgemäß verwendeten Mitteln um Produkte, die nach einer bestimmten Einwirkzeit, in der Regel ca. 1 Sekunde bis 1 Stunde, wieder aus dem Haar ausgespült werden. Im Rahmen dieser Ausführungsform sind Haarshampoos und Haarspülungen ganz besonders bevorzugt.

Bei auf dem Haar, zumindest bis zur nächsten Haarwäsche, verbleibenden Produkten sind Haarkuren besonders bevorzugte erfindungsgemäße Zubereitungen.

Die erfindungsgemäß verwendeten Shampoos enthalten in der Regel mindestens eine reinigende Komponente; diese wird üblicherweise aus der Gruppe der anionischen, ampholytische, zwitterionischen und nichtionogenen Tenside ausgewählt; es kann sich aber auch gemäß einer weiteren Ausführungsform um kationische Tenside handeln.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium-, Magnesium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Amidethercarboxylate der Formel [R-NH(-CH₂-CH₂-O)ₙ-CH₂-COO]ₘZ, in der R für einen linearen oder verzweigten, gesättigten oder ungesättigten Acylrest mit 2 bis 29 C-Atomen, n für ganze Zahlen von 1 bis 10, m für die Zahlen 1 oder 2 und Z für ein Kation aus der Gruppe der Alkali- oder Erdalkalimetalle steht,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Kokosmonoglyceridsulfate.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel RO-(Z)ₓ. Diese Verbindungen sind durch die folgenden Parameter gekennzeichnet.

Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung der Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können, insbesondere als Co-Tenside, zwitterionische Tenside verwendet werden. Als zwitterionische Tenside werden solche oberflächenaktiven Verbindungen bezeichnet, die im Molekül mindestens- eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyl-dimethylammonium-glycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Beispiele für die in den erfindungsgemäß verwendeten Haarbehandlungsmitteln verwendbaren kationischen Tenside sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex® vertriebenen Dialkylammoniummethosulfate und Methyl-hydroxyalkyl-dialkoyloxyalkyl-ammoniummethosulfate und die entsprechenden Produkte der Dehyquart®-Reihe, als kationische Tenside eingesetzt werden. Weitere erfindungsgemäß verwendbare kationische Tenside stellen die quaternisierten Proteinhydrolysate dar.

Vorzugsweise enthalten die erfindungsgemäß verwendeten Shampoos die oberflächenaktiven Verbindungen in Mengen von 5,0 bis 40 Gew.-%, insbesondere von 5,0 bis 20 Gew.-%, bezogen auf die jeweilige Zubereitung.

Soll das Shampoo neben der gewünschten Reinigungsleistung insbesondere eine große Mildheit gegenüber der Haut aufweisen, wie es beispielsweise für Baby- oder Kindershampoos gewünscht wird, so wird man gemäß einer bevorzugten Ausführungsform als Tensidbasis aus anionischen und zwitterionischen Tensiden auswählen. Das anionische Tensid wird man speziell aus Alkylethersulfaten und Ethercarbonsäuresalzen auswählen, das zwitterionische Tenside aus den Betainen. Tensidmischungen, bei denen das Mengen-Verhältnis von anionischen Tensiden zu zwitterionischen Tensiden im Bereich von 2:1 bis 1:2, insbesondere von 1,5:1 bis 1:1 liegt, sind besonders geeignet.

Gemäß einer weiteren Ausführungsform wird man zur Formulierung besonders milder Shampoo ein Niotensid vom Typ der Alkylglykoside als reinigende Komponente auswählen. Auch die Dreierkombinationen aus den bevorzugten anionischen und zwitterionischen Tensiden und den Alkylglykosiden haben sich als exzellente Tensidgrundlagen erwiesen, vorzugsweise mit einem Mengenverhältnis Aniontensid : zwitterionisches Tensid : Alkylglykosid von 1-4 : 1-3 : 0.5-2, wobei die Menge an Aniontensid und zwitterionischem Tensid jeweils mindestens das 1,5fache der Menge des Alkylglykosid betrug.

Gemäß einer weiteren bevorzugten Ausführungsform enthalten die erfindungsgemäß verwendeten Zubereitungen, insbesondere Shampoos, mindestens einen konditionierenden Wirkstoff. Dadurch wird insbesondere auch die Kämmbarkeit der Haare nach der Behandlung verbessert. Dies ist speziell für Shampoos zu empfehlen, die für die Zielgruppe "Kinder" formuliert werden, um der bekannten Empfindlichkeit der Kinder beim Kämmen insbesondere des nassen Haares zu entsprechen.

Als konditionierende Wirkstoffe kommen bevorzugt kationische Polymere in Betracht. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- Polysiloxane mit quaternären Gruppen,
- Polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammo-niumchlorid-Acrylamid-Copolymer) im Handel erhältlichen Produkte sind Beispiele für solche kationischen Polymere.
- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Geeignet als konditionierende Wirkstoffe sind auch Ampho-Polymere. Unter dem Oberbegriff Ampho-Polymere sind amphotere Polymere, d.h. Polymere, die im Molekül sowohl freie Aminogruppen als auch freie -COOH- oder SO₃H-Gruppen enthalten und zur Ausbildung innerer Salze befähigt sind, zwitterionische Polymere, die im Molekül quartäre Ammoniumgruppen und -COO⁻- oder -SO₃⁻-Gruppen enthalten, und solche Polymeren zusammengefaßt, die -COOH- oder SO₃H-Gruppen und quartäre Ammoniumgruppen enthalten. Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt. Ebenfalls bevorzugte Amphopolymere setzen sich aus ungesättigten Carbonsäure (z.B. Acryl- und Methacryl-säure), kationisch derivatisierten ungesättigten Carbonsäure (z.B. Acrylamidopropyl-trimethyl-ammoniumchlorid) und gegebenenfalls weiteren ionischen oder nichtionogenen Monomeren zusammen, wie beispielsweise in der deutschen Offenlegungsschft 39 29 973 und dem dort zitierten Stand der Technik zu entnehmen sind. Die kationischen oder amphoteren Polymere sind in den erfindungsgemäßen Zubereitungen bevorzugt in Mengen von 0,1 bis 5 Gew.-%, bezogen auf die gesamte Zubereitung, enthalten.

Proteinhydrolysate, insbesondere Elastin-, Kollagen-, Keratin-, Milcheiweiß-, Sojaprotein-, Mandelprotein-, Erbsenprotein-, Kartoffelprotein-, Haferprotein-, Maisprotein- und Weizenproteinhydrolysate, deren Kondensationsprodukte mit Fettsäuren sowie quaternisierte Proteinhydrolysate sind ebenfalls als konditionierende Wirkstoffe geeignet. Dabei können Produkte auf pflanzlicher Basis erfindungsgemäß bevorzugt sein.

Als konditionierende Wirkstoffe geeignete Silikonöle sind insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200 und DC 1401 vertriebenen Produkte.

Erfindungsgemäß als konditionierende Wirkstoffe ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 7232 (Hersteller: Th. Goldschmidt; diquatemäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Dialkyl-ammoniummethosulfate sowie die entsprechenden Produkte der Dehyquart®-Serie.

Bevorzugte pflanzliche Öle als konditionierende Wirkstoffe sind Jojobaöl, Sonnenblumenöl, Orangenöl, Mandelöl, Weizenkeimöl und Pfirsichkernöl. Jojobaöl und Orangenöl sind besonders bevorzugt.

Insbesondere für die Formulierung sehr milder Zubereitungen hat es sich weiterhin als vorteilhaft erwiesen, wenn die Menge an gelösten anorganischen Salzen auf weniger als 2 Gew.-%, insbesondere weniger als 0,5 Gew.-% begrenzt wird. Dabei ist auch zu beachten, daß solche Salze nicht nur z.B. zur Einstellung der Viskosität zugegeben werden, sondern auch durch andere Wirkstoffe, insbesondere Tenside, eingebracht werden können.

Weiterhin kann es bevorzugt sein, die erfindungsgemäß verwendeten Zubereitungen so zu formulieren, daß ihre bestimmungsgemäße Haltbarkeit ohne den Zusatz von Konservierungsmitteln gewährleistet werden kann. Sollte die Verwendung von Konservierungsmitteln notwendig sei, so ist es bevorzugt, solche Konservierungsmittel einzusetzen, die auch für die Konservierung von Lebensmitteln verwendet werden. Beispiele hierfür sind Benzoesäure und Sorbinsäure sowie deren Alkali-, insbesondere Natriumsalze.

Entsprechend dem Verwendungszweck und der Formulierungsart können die erfindungsgemäß verwendeten Zubereitungen alle für den jeweiligen Verwendungszweck üblichen kosmetischen Zusätze enthalten.

Solche üblichen Bestandteile sind:
- nichtionische Polymere wie beispielsweise Vinylpyrrolidon/Vinylacrylat-Copolymere, Polyvinylpyrrolidon und Vinylpyrrolidon/Vinylacetat-Copolymere,
- anionische Polymere, wie Polyacryl- und Polymethacrylsäuren, deren Salze, deren Copolymere mit Acrylsäure- und Methacrylsäureestern und -amiden und deren Derivate, die durch Kreuzvernetzung mit polyfunktionellen Agentien erhalten werden,
   Polyoxycarbonsäuren, wie Polyketo- und Polyaldehydocarbonsäuren und deren Salze,
   sowie Polymere und Copolymere der Crotonsäure mit Estern und Amiden der Acryl- und der Methacrylsäure, wie Vinylacetat-Crotonsäure- und Vinylacetat-Vinylpropionat-Crotonsäure-Copolymere,
- Verdickungsmittel wie Agar-Agar, Guar-Gum, Alginate, Celluloseether, Gelatine, Pektine und/oder Xanthan-Gum,
- Strukturanten wie Glucose und Maleinsäure,
- haarkonditionierende Verbindungen-wie Phospholipide, beispielsweise Sojalecithin, Ei-Lecithin und Kephaline,
- weitere Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Climbazol, Piroctone Olamine und Zink Omadine,
- Wirkstoffe wie Bisabolol, Allantoin und Pflanzenextrakte,
- Lichtschutzmittel,
- Konsistenzgeber wie Zuckerester, Polyolester oder Polyolalkylether,
- Fette und Wachse, wie Walrat, Bienenwachs, Montanwachs, Paraffine, Ester, Glyceride und Fettalkohole,
- Fettsäurealkanolamide,
- Komplexbildner wie EDTA, NTA, β-Alanindiessigsäure und Phosphonsäuren,
- Quell- und Penetrationsstoffe wie PCA, Glycerin, Propylenglykolmonoethylether, Carbonate, Hydrogencarbonate, Guanidine, Harnstoffe sowie primäre, sekundäre und tertiäre Phosphate,
- Trübungsmittel wie Latex oder Styrol/Acrylamid-Copolymere,
- Perlglanzmittel wie Ethylenglykolmono- und -distearat oder PEG-3-distearat,
- direktziehende Farbstoffe
- sogenannte Kuppler- und Entwicklerkomponenten als Oxidationsfarbstoffvorprodukte,
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat,
- Treibmittel wie Propan-Butan-Gemische, N₂O, Dimethylether, CO₂ und Luft sowie
- Antioxidantien.

Der pH-Wert der erfindungsgemäß verwendeten Zubereitungen liegt üblicherweise bei 2,5 bis 10. Insbesondere erfindungsgemäße Haarshampoos und -spülungen sowie Wellotionen weisen bevorzugt einen pH-Wert von 2,5 bis 7,0, insbesondere von 4,0 bis 6,0, auf. Dagegen liegt der pH-Wert von erfindungsgemäß verwendeten Oxidationsfärbemitteln in der Regel bei 7,0 bis 10,0, insbesondere bei 7,0 bis 9,0.

Zur Einstellung dieses pH-Wertes kann praktisch jede für kosmetische Zwecke verwendbare Säure oder Base eingesetzt werden. Für den Fall, daß zur pH-Wert-Einstellung eine Säure verwendet wird, kann es bevorzugt sein, eine Säure aus der Gruppe der Genußsäuren wie beispielsweise Essigsäure, Milchsäure, Weinsäure, Zitronensäure, Äpfelsäure, Ascorbinsäure und Gluconsäure zu verwenden. Im Rahmen der Erfindung ist die Verwendung von Zitronensäure und Milchsäure besonders bevorzugt.

### Beispiele

Alle Mengenangaben sind, soweit nicht anders vermerkt, Gewichtsteile.

### 1. Haarshampoo

| | |
|---|---|
| Texapon® N25¹ | 30,0 |
| Dehyton® PK-45² | 9,0 |
| Plantacare® 818³ | 2,0 |
| Polymer JR® 400⁴ | 0,1 |
| Arlypon® F⁵ | 0,5 |
| Cetiol® HE⁶ | 0,5 |
| Bitrex®⁷ | 0,003 |
| Natriumchlorid | 0,1 |
| Parfümöl Duftnote Cola | q.s. |
| oder | |
| Parfümöl Duftnote Erdbeere | q.s. |
| Konservierungsmittel | q.s. |
| Zitronensäure | ad pH 5,5 |
| Wasser | ad 100 |

| | |
|---|---|
| ¹ Natriumlaurylethersulfat (ca. 28 % Aktivsubstanz in Wasser; INCI-Bezeichnung: Sodium Laureth Sulfat) (HENKEL) | |
| ² Fettsäureamidderivat mit Betain-Struktur (ca. 47 % Aktivsubstanz in Wasser; INCI-Bezeichnung: Cocamidopropyl Betaine) (HENKEL) | |
| ³ C8-14-Alkylpolyglucosid (ca. 52 % Aktivsubstanz in Wasser; INCI-Bezeichnung: Coco Glucoside) (HENKEL) | |
| ⁴ quaternierte Hydroxyethylcellulose (INCI-Bezeichnung: Polyquaternium-10) AMERCHOL) | |
| ⁵ C12-14-Fettalkohol + 2,5 Ethylenoxid (INCI-Bezeichnung: Laureth-2) (HENKEL) | |
| ⁶ Kokosmonoglycerid + 7,3 Ethylenoxid (INCI-Bezeichnung: PEG-7 Glyceryl Cocoate) (HENKEL) | |
| ⁷ Benzyldiethyl((2,6-xylylcarbamoyl)methyl)-ammoniumbenzoat (MACFARLAN SMITH) | |

### 2. Haarshampoo

| | |
|---|---|
| Genapol®LRO⁸ | 10,0 |
| Rewoteric®AM2 CNM⁹ | 6,0 |
| Merquat®550¹⁰ oder | 1,5 |
| Luviquat® FC 550¹¹ oder | 0,5 |
| Gafquat® 734¹² | 1,0 |
| Antil®141¹³ | 0,2 |
| Indigestin® ¹⁴ | 0,002 |
| Jojobaöl | 0,2 |
| Cremophor®RH 455¹⁵ | 0,2 |
| Natriumchlorid | 0,3 |
| Parfümöl Duftnote Pfirsich | q.s. |
| Wasser | ad 100 |

| | |
|---|---|
| ⁸ C12-14-Alkohol + 2 Ethylenoxid-sulfat, Natriumsalz (ca. 69 % Aktivsubstanz in Wasser; INCI-Bezeichnung: Sodium Laureth Sulfate) (HOECHST) | |
| ⁹ N-Kokosfettsäureamidoethyl-N-2-hydroxyethylglycin-Natriumsalz (ca. 50 % Aktivsubstanz in Wasser; INCI.-Bezeichnung: Disodium Cocoamphodiacetate) (WITCO) | |
| ¹⁰ Dimethyldiallylammoniumchlorid-Acrylamid-Copolymer (ca. 9 % Aktivsubstanz in Wasser; INCI-Bezeichnung: Polyquaternium-7) (MERCK) | |
| ¹¹ Vinylimidazoliummethochlorid-Vinylpyrrolidon-Copolymerisat (50:50) (ca. 40 % Aktivsubstanz in Wasser; INCI-Bezeichnung: Polyquaternium-16) (BASF) | |
| ¹² Dimethylaminoethylmethacrylat-Vinylpyrrolidon-Copolymer, quaterniert mit Diethylsulfat (ca. 19 % Aktivsubstanz in Wasser; INCI-Bezeichnung: Polyquaternium-11) (GAF) | |
| ¹³ Polyoxyethylen-propylenglykol-dioleat (INCI-Bezeichnung: PEG-55 Propylene Glycol Oleate) (GOLDSCHMIDT) | |
| ¹⁴ Benzyldiethyl((2,6-xylylcarbamoyl)methyl)-ammoniumbenzoat (CHASSOT) | |
| ¹⁵ hydriertes Rizinusöl + 45 Ethylenoxid (ca. 90 % Aktivsubstanz; INCI-Bezeichnung: PEG-40 Hydrogenated Castor Oil (and) Propylene Glycol) (BASF) | |

### 3. Haar-Spülung

| | |
|---|---|
| Tegoamid®S 18¹⁶ | 1,0 |
| Dehyquart®F-75¹⁷ | 0,5 |
| Lanette®O¹⁸ | 3,5 |
| Phenoxyethanol | 0,9 |
| Bitrex® | 0,004 |
| Parfümöl Duftnote Rum | q.s. |
| Citronensäure | ad pH 4,5 |
| Wasser | ad 100 |

| | |
|---|---|
| ¹⁶ N,N-Dimethyl-N'-stearoyl-1,3-diaminopropan (INCI-Bezeichnung: Stearamidopropyl Dimethylamine) (GOLDSCHMIDT) | |
| ¹⁷ Fettalkohole-Methyltriethanolammoniummethylsulfatdialkylester-Gemisch (INCI-Bezeichnung: Distearoylethyl Hydroxyethylmonium Methosulfate (and) Cetearyl Alcohol) (HENKEL) | |
| ¹⁸ C16-18-Fettalkohol (INCI-Bezeichnung: Cetearyl Alcohol) (HENKEL) | |

### 4. Haarkur (leave-on)

| | |
|---|---|
| Ethanol | 10,0 |
| Dehyquart® SP¹⁹ | 0,1 |
| Promois® WK²⁰ | 0,5 |
| Uvinul®MS 40²¹ | 0,1 |
| Parfümöl Duftnote Melone oder | q.s. |
| Parfümöl Duftnote Apfel | q.s. |
| Bitrex® | 0,0015 |
| Wasser | ad 100 |

| | |
|---|---|
| ¹⁹ Oxyethylalkylammoniumphosphat (ca. 50 % Aktivsubstanz in Wasser; INCI-Bezeichnung: Quaternium 52) (HENKEL) | |
| ²⁰ Keratinhydrolysat (ca. 25 % Aktivsubstanz in Wasser) (SEIWA) | |
| ²¹ 2-Hydroxy-4-methoxybenzophenon-5-sulfonsäure (INCI-Bezeichnung: Benzophenone-4) (BASF) | |

## Patentansprüche

1. Verwendung einer wässrigen Zubereitung zur Behandlung von Keratinfasern, insbesondere menschlichen Haaren, enthaltend mindestens einen natürlichen oder synthetischen Inhaltsstoff mit der Duftnote eines Lebens- oder Genußmittels, **dadurch gekennzeichnet, dass** die Zubereitung weiterhin 0,0005 bis 0,1 Gew.-% eines Bitterstoffs enthält, der eine Molmasse von mindestens 250 g/mol aufweist und in der Zubereitung bei 20 °C zu mindestens 10 mg/l löslich ist.

2. Verwendung nach Anspruch 1, **dadurch gekennzeichnet, dass** der Bitterstoff in Wasser bei 20 °C zu mindestens 5 g/l löslich ist.

3. Verwendung nach Anspruch 1 oder 2, **dadurch gekennzeichnet, dass** der Bitterstoff ionogen ist.

4. Verwendung nach Anspruch 3, **dadurch gekennzeichnet, dass** der Bitterstoff eine quartäre Ammoniumverbindung ist.

5. Verwendung nach Anspruch 4, **dadurch gekennzeichnet, dass** der Bitterstoff Benzyldiethyl(2,6-xylylcarbamoyl)methyl)ammoniumbenzoat ist.

6. Verwendung nach einem der Ansprüche 1 bis 5, **dadurch gekennzeichnet, dass** der Inhaltsstoff mit der Duftnote eines Lebens- oder Genußmittels ein Parfümöl ist.

7. Verwendung nach Anspruch 6, **dadurch gekennzeichnet, dass** das parfümöl die Duftnote einer Frucht aufweist.

8. Verwendung nach Anspruch 7, **dadurch gekennzeichnet, dass** die Frucht ausgewählt ist aus Apfel, Birne, Erdbeere, Pfirsich, Aprikose, Ananas, Banane, Kirsche, Kiwi Mango, Kokos Mandel, Grapefruit, Maracuja, Mandarine und Melone.

9. Verwendung nach Anspruch 6, **dadurch gekennzeichnet, dass** das Parfümöl die Duftnote eines Genußmittels aufweist.

10. Verwendung nach Anspruch 9, **dadurch gekennzeichnet, dass** das Genußmittel ausgewählt ist aus Tabak, Cola, Guarana, Schokolade, Kakao, Pfefferminze und Rum.

11. Verwendung nach einem der Anspruche 1 bis 10, **dadurch gekennzeichnet, dass** die Zubereitung zur Behandlung von Keratinfasern dazu bestimmt ist, nach einer Einwirkzeit von 1 Sekunde bis 1 Stunde von der keratinischen Faser wieder abgespült zu werden.

12. Verwendung nach einem der Ansprüche 1 bis 11, **dadurch gekennzeichnet, dass** die Zubereitung zur Behandlung von Keratinfasern weiterhin ein mildes Tensid oder eine milde Tensidmischung enthält.

13. Verwendung nach Anspruch 12, **dadurch gekennzeichnet, dass** die Zubereitung zur Behandlung von Keratinfasern ein Alkylgykosid enthält.

14. Verwendung nach Anspruch 12 oder 13, **dadurch gekennzeichnet, dass** die Zubereitung zur Behandlung von Keratinfasern eine Mischung aus einem anionischen Tensid und einem zwitterionischen Tensid enthält.

15. Verwendung nach einem der Ansprüche 1 bis 14, **dadurch gekennzeichnet, dass** die Zubereitung zur Behandlung von Keratinfasern einen konditionierenden Wirkstoff enthält.

16. Verwendung nach Anspruch 15, **dadurch gekennzeichnet, dass** der konditionierende Wirkstoff ausgewählt ist aus kationischen Polymeren, Ampho-Polymeren, Proteinhydrolysaten, Derivaten von Proteinhydrolysaten, Silikonölen und pflanzlichen Ölen.

17. Verwendung nach einem der Ansprüche 1 bis 16, **dadurch gekennzeichnet, dass** die Zubereitung zu Behandlung von Keratinfasern weniger als 2,0 Gew.-% an gelösten anorganischen Salzen enthält.

18. Verwendung nach einem der Ansprüche 1 bis 17, **dadurch gekennzeichnet, dass** die Zubereitung zur Behandlung von Keratinfasern frei von Konservierungsmitteln ist, die nicht für den Gebrauch in Lebensmitteln zugelassen sind.

## Claims

1. Use of an aqueous composition for treating keratin fibres, in particular human hair, comprising at least one natural or synthetic ingredient with the scent note of a foodstuff or luxury product, **characterized in that** the composition also comprises 0.0005 to 0.1% by weight of a bitter substance which has a molar mass of at least 250 g/mol and is soluble in the composition at 20°C to at least 10 mg/l.

2. Use according to Claim 1, **characterized in that** the bitter substance is soluble in water at 20°C to at least 5 g/l.

3. Use according to Claim 1 or 2, **characterized in that** the bitter substance is ionogenic.

4. Use according to Claim 3, **characterized in that** the bitter substance is a quaternary ammonium compound.

5. Use according to Claim 4, **characterized in that** the bitter substance is benzyldiethyl(2,6-xylylcarbamoyl)methyl)ammonium benzoate.

6. Use according to one of Claims 1 to 5,
**characterized in that** the ingredient with the scent note of a foodstuff or luxury product is a perfume oil.

7. Use according to Claim 6, **characterized in that** the perfume oil has the scent note of a fruit.

8. Use according to Claim 7, **characterized in that** the fruit is chosen from apple, pear, strawberry, peach, apricot, pineapple, banana, cherry, kiwi, mango, coconut, almond, grapefruit, passion fruit, mandarin and melon.

9. Use according to Claim 6, **characterized in that** the perfume oil has the scent note of a luxury product.

10. Use according to Claim 9, **characterized in that** the luxury product is chosen from tobacco, cola, guarana, chocolate, cocoa, peppermint and rum.

11. Use according to one of Claims 1 to 10, **characterized in that** the composition for treating keratin fibres is intended to be rinsed off again from the keratin fibre after a contact time of from 1 second to 1 hour.

12. Use according to one of Claims 1 to 11, **characterized in that** the composition for treating keratin fibres also comprises a mild surfactant or a mild surfactant mixture.

13. Use according to Claim 12, **characterized in that** the composition for treating keratin fibres comprises an alkyl glycoside.

14. Use according to Claim 12 or 13, **characterized in that** the composition for treating keratin fibres comprises a mixture of an anionic surfactant and a zwitterionic surfactant.

15. Use according to one of Claims 1 to 14, **characterized in that** the composition for treating keratin fibres comprises a conditioning active ingredient.

16. Use according to Claim 15, **characterized in that** the conditioning active ingredient is chosen from cationic polymers, ampho polymers, protein hydrolysates, derivatives of protein hydrolysates, silicone oils and vegetable oils.

17. Use according to one of Claims 1 to 16, **characterized in that** the composition for treating keratin fibres comprises less than 2.0% by weight of dissolved inorganic salts.

18. Use according to one of Claims 1 to 17, **characterized in that** the composition for treating keratin fibres is free from preservatives which are not approved for use in foodstuffs.

## Revendications

1. Utilisation d'une préparation aqueuse pour traiter des fibres kératiniques, en particulier des cheveux humains, contenant au moins un ingrédient naturel ou synthétique, ayant la note de parfum d'un produit alimentaire ou stimulant, **caractérisée en ce que** la préparation contient en plus 0,0005 à 0,1% en poids d'une substance amère, qui présente une masse molaire d'au moins 250 g/mole et qui est soluble dans la préparation à 20°C à raison d'au moins 10 mg/l.

2. Utilisation selon la revendication 1, **caractérisée en ce que** la substance amère est soluble dans l'eau à 20°C à raison d'au moins 5 g/l.

3. Utilisation selon la revendication 1 ou 2, **caractérisée en ce que** la substance amère est ionogène.

4. Utilisation selon la revendication 3, **caractérisée en ce que** la substance amère est un composé d'ammonium quaternaire.

5. Utilisation selon la revendication 4, **caractérisée en ce que** la substance amère est le benzoate de benzyl(diéthyl(2,6-xylyl-carbamoyl)-méthyl)ammonium.

6. Utilisation selon l'une quelconque des revendications 1 à 5, **caractérisée en ce que** l'ingrédient ayant la note de parfum d'un produit alimentaire ou stimulant, est une huile de parfum.

7. Utilisation selon la revendication 6, **caractérisée en ce que** l'huile de parfum présente la note de parfum d'un fruit.

8. Utilisation selon la revendication 7, **caractérisée en ce que** le fruit est choisi parmi la pomme, la poire, la fraise, la pêche, l'abricot, l'ananas, la banane, la cerise, le kiwi, la mangue, la noix de coco, l'amande, le pamplemousse, la maracuja, la mandarine, et le melon.

9. Utilisation selon la revendication 6, **caractérisée en ce que** l'huile de parfum présente la note de parfum d'un produit stimulant.

10. Utilisation selon la revendication 9, **caractérisée en ce que** le produit stimulant est choisi parmi le tabac, le cola, le guarana, le chocolat, le cacao, la menthe poivrée et le rhum.

11. Utilisation selon l'une quelconque des revendications 1 à 10, **caractérisée en ce que** la préparation pour traiter les fibres kératiniques est destinée à être de nouveau rincée des fibres kératiniques, après une durée d'action d'une seconde à 1 heure.

12. Utilisation selon l'une quelconque des revendications 1 à 11, **caractérisée en ce que** la préparation pour traiter les fibres kératiniques contient en sus un tensio-actif doux ou un mélange tensio-actif doux.

13. Utilisation selon la revendication 12, **caractérisée en ce que** la préparation pour traiter les fibres kératiniques contient un glycoside d'alkyle.

14. Utilisation selon la revendication 12 ou 13, **caractérisée en ce que** la préparation pour traiter les fibres kératiniques contient un mélange d'un tensio-actif anionique et d'un tensio-actif zwitterionique.

15. Utilisation selon l'une quelconque des revendications 1 à 14, **caractérisée en ce que** la préparation pour traiter les fibres kératiniques contient une substance active de conditionnement.

16. Utilisation selon la revendication 15, **caractérisée en ce que** la substance active de conditionnement est choisie parmi les polymères cationiques, les amphopolymères, les hydrolysats de protéines, les dérivés d'hydrolysats de protéines, les huiles de silicone et les huiles végétales.

17. Utilisation selon l'une quelconque des revendications 1 à 16, **caractérisée en ce que** la préparation pour traiter les fibres kératiniques contient moins de 2,0% en poids de sels minéraux dissous.

18. Utilisation selon l'une quelconque des revendications 1 à 17, **caractérisée en ce que** la préparation pour traiter les fibres kératiniques est exempte d'agents conservateurs qui ne sont pas autorisés pour une utilisation dans les denrées alimentaires.
